Europäisches Patentamt

European Patent Office

Office européen des brevets

(19)

(11) Veröffentlichungsnummer: **0 204 273**

**A2**

(12) **EUROPÄISCHE PATENTANMELDUNG**

(21) Anmeldenummer: **86107375.7**

(51) Int. Cl.4: **C02F 3/30**

(22) Anmeldetag: **30.05.86**

(30) Priorität: **05.06.85 DE 3520160**

(43) Veröffentlichungstag der Anmeldung:
**10.12.86 Patentblatt 86/50**

(84) Benannte Vertragsstaaten:
**BE CH FR GB IT LI NL**

(71) Anmelder: **Noell GmbH**
**Alfred-Nobel-Strasse 20**
**D-8700 Würzburg(DE)**

(72) Erfinder: **Menzel, Roman, Dr. Dr.-Ing.**
**Quellenweg 10**
**D-4450 Lingen 1(DE)**
Erfinder: **Hoffmann, Peter**
**Meppener Strasse 70i**
**D-4450 Lingen 1(DE)**
Erfinder: **Vorlop, Klaus-Dieter, Dr.**
**Dipl.-Biochem.**
**Hochstrasse 7**
**D-3300 Braunschweig(DE)**

(74) Vertreter: **König, Reimar, Dr.-Ing. et al**
**Patentanwälte Dr.-Ing. Reimar König**
**Dipl.-Ing. Klaus Bergen Wilhelm-Tell-Strasse**
**14 Postfach 260162**
**D-4000 Düsseldorf 1(DE)**

(54) **Verfahren und Vorrichtung zum Entfernen von Nitrat aus Oberflächen- und Grundwasser, insbesondere Trinkwasser.**

(57) Verfahren zum Entfernen von Nitrat aus Oberflächen-und Grundwasser, insbesondere Trinkwasser, mit Hilfe der biologischen Dentrifikation bei der in einem Reaktor (9, 16) mit Hilfe eines Biokatalysators (12, 19) mit immobilisierten Zellen, die in einem Gel oder Polymer vernetzt sind, die im Wasser enthaltenen Nitrate zu gasförmigem Stickstoff reduziert werden. Der vorzugsweise verwendete Stamm "Micrococcus denitrificans" benötigt für seinen Stoffwechsel Wasserstoff, der zudosiert wird und Kohlenstoff, der im Wasser in Form von gelöstem Kohlendioxid vorhanden ist. Durch die Verwendung eines Biokatalysators aus immobilisierten Zellen und dem verwendeten Bakterienstamm ist keine aufwendige Nachreinigung aufgrund erhöhter Bakterienzahlen im Reinwasserablauf erforderlich, und es entstehen keine unerwünschten Nebenprodukte.
.

**"Verfahren und Vorrichtung zum Entfernen von Nitrat aus Oberflächen-und Grundwasser, insbesondere Trinkwasser"**

Die Erfindung betrifft ein Verfahren und eine Vorrichtung zum Entfernen von Nitrat aus Oberflächen-und Grundwasser, insbesondere Trinkwasser, mit Hilfe der biologischen Denitrifikation.

In vielen Wasserversorgungsunternehmen ist im Laufe der letzten Jahre ein starker Anstieg des Nitratgehaltes über 50 mg/l $NO_3^-$/l zu verzeichnen, der seine Ursachen u.a. in der Überdüngung landwirtschaftlicher Nutzflächen und unzweckmäßiger Entsorgung der tierischen Abwässer hat. Nach Bekanntwerden von Zusammenhängen zwischen hohen Nitratwerten und z.B. Krebs der Verdauungsorgane wurden für 1985 durch die europäische Gemeinschaft neue Richtwerte erarbeitet, die für Nitrat eine Richtzahl von 25 mg/l $NO_3^-$/l und eine zulässige Höchskonzentration von weniger als 50 mg/l $NO_3^-$/l im Trinkwasser zulassen. In vielen Fällen lassen sich daher Aufbereitungsmaßnahmen nicht vermeiden, um niedrigere Nitratkonzentrationen bei der Trinkwasserversorgung sicher zu stellen.

In einem Aufsatz "Entfernen von Nitrat aus dem Trinkwasser mit Hilfe der biologischen Denitrifikation" von Ch.Frank und W. Dott, Bonn, erschienen in BTF 3/4 84, S.53 bis 57, sind Untersuchungen derartiger biologischer Verfahren beschrieben. Bei diesen Verfahren werden Bakterien auf den verschiedensten Trägern wie offenporigen Strukturen mit Aufwuchsmaterial, Tropfkörper, Tauchkörper, Bims, Aktivkohle, Styroporkugeln, Schaumstoff usw. fixiert und in entsprechenden Reaktoren in der Wasser-und Abwasserreinigung eingesetzt.

Man unterscheidet zwischen zwei unterschiedlichen biologischen Verfahren:

Die lithotrophe, (auch genannt autotrophe) Denitrifikation, bei der die Zugabe von Wasserstoff oder elementarem Schwefel als Elektronendonator notwendig ist. Als Kohlenstoffquelle dient hier die im Wasser vorhandene, gelöste Kohlensäure.

Die organotrophe (auch genannt heterotrophe) Denitifrikation, bei der die Zugabe eines Kohlenstoffträgers, wie z.B. Methyl-oder Äthylalkohol, Zucker, Melasse, Essigsäure, Methan oder Erdgas erforderlich ist.

Bei den bisher in der Praxis eingesetzten biologischen Verfahren wird wegen der günstigeren Reaktionszeit die Zudosierung von Methanol oder Äthanol als Kohlenstoffträger bevorzugt. Bei dieser Konzeption können sich unter bestimmten Betriebszuständen (z.B. Überdosierung oder zu kurze Reaktionszeit) unerwünschte Nebenprodukte wie z.B. Formaldehyd und Acetaldehyd bilden, die nach den MAK-Wert-Richtlinien als Stoff mit begründetem Verdacht auf Krebs erzeu gendes Potential eingestuft werden. Daher verlangen solche Verfahrenskonzepte ein aufwendiges Regel-und Überwachungssystem, das eine zusätzliche Störquelle darstellt.

Setzt man stattdessen andere Kohlenstoffträger, wie z.B. Zucker, Melasse oder Essigsäure ein, so entstehen nach heutigem Kenntnisstand zwar keine verdächtigen Reaktionsprodukte, jedoch benötigen solche Verfahren infolge langer Reaktionszeiten große Reaktorvolumina, die einen erheblichen Apparate-und Investitionsaufwand bedeuten.

Des weiteren bestehen alle bekannten, für die Denitrifikation eingesetzten Trägersysteme für die denitrifizierenden Bakterien aus Kontaktmassen in offenporiger Struktur und sind daher gegenüber Giftstoffen, wie z.B. Metallionen (Chrom, Eisen, Kupfer, etc.) anfällig und benötigen eine zusätzliche Vorbehandlungsstufe.

Schließlich führt die verfahrensbedingte Zudosierung einer Kohlenstoffquelle zum Wasser zwangsläufig zu einer Vermehrung der im Wasser vorhandenen Bakterien. Daher sind derartige Verfahren nicht nur an der Verminderung der Nitratbelastung zu messen, sondern es muss auch die veränderte bakteriologische Situation im abgegebenen Reinwasser sowie die zwingend nötige Nachaufbereitung durch z.B. Mehrschichtfilteranlagen berücksichtigt werden. Dabei steht neben dem quantitativen Effekt, den Bakterienzahlen, auch die Frage nach den vorkommenden Arten im Vordergrund. So wurde festgestellt, daß nach dem Denitrifikationsreaktor im Wasser Bakterienzahlen in einer Größenordnung von $10^4$ bis $10^5$/ml vorlagen, die sich erst durch die Nachbehandlung z.B. Filtration mit Zwischenbelüftung auf die im Reinwasser übliche Größenordnung von $10^2$ bis $10^3$/ml reduzierten.

Der Erfindung liegt die Aufgabe zugrunde, ein Verfahren und eine Vorrichtung der eingangs erwähnten Art zu schaffen, die kurze Reaktionszeiten sowie geringen apparativen Aufwand erfordern und keine unerwünschten Nebenprodukte und keine Vergrößerung der Bakterienanzahl und -arten im Reinwasser entstehen lassen.

Gelöst wird diese Aufgabe durch die Verwendung eines Biokatalysators mit immobilisierten Zellen, die in einem Gel oder Polymer vernetzt sind, vorzugsweise aus einem Kern mit im vernetzten Gel oder Polymer immobilisierten Zellen und einer zellenfreien, aus einem vernetzten, keine Durchlässigkeit für Zellen aufweisenden Gel oder Polymer bestehenden, den zellenhaltigen Kern umschließenden Schutzschicht.

Ein derartiger Biokatalysator ist in der nicht vorveröffentlichten deutschen Patentanmeldung P 34 32 923.4 mit Bezug auf die Herstellung von Antibiotika oder Äthanol, die Abwasserreinigung oder für die Sektherstellung beschrieben.

Durch die erfindungsgemäße Verwendung eines derartigen Biokatalysators ergibt sich eine erhöhte Biomassenkonzentration im Katalysatorbett, wodurch in kürzester Zeit ein Nitratabbau erreicht wird. Gegenüber Trägersystemem mit offenporiger Struktur liegen die erreichbaren Verbesserungen bezüglich der Reaktionszeit und des apparativen Aufwandes bei mehr als 60%.

Nach längerem Einsatz kann der Biokatalysator, falls erforderlich, ohne Schwierigkeiten ausgetauscht werden. Dies wird zusätzlich dadurch erleichtert, daß der Biokatalysator in getrockneter Form gelagert werden kann und sofort verfügbar ist und daß es auch keine Schwierigkeiten bereitet, vor Ort eine kurzfristige Eigenproduktion einzurichten. Da der inaktive Biokatalysator in jeder Hinsicht unbedenklich ist, ist keine chemische Sonderbehandlung vor der Entsorgung durch Ableitung in die Kanalisation oder Kläranlage notwendig.

Durch kurzfristig auftretende toxische Begleitstoffe im zu denitrifizierenden Wasser, wie z.B. Metallionen, deren Konzentration nicht nennenswert über derjenigen des Kalziums im Rohwasser liegt, erfolgt keine negative Beeinflussung.

Des weiteren ist eine Ansiedlung von unterschiedlichen Bakterienkulturen und eine Vermehrung der Bakterienanzahl, wie dies bei Trägersystemen mit offenporigen Strukturen eintreten kann, ausgeschlossen. Aus diesem Grunde ist keine aufwendige Nachreinigung, z.B. durch Mehrschichtfiltration, um die Bakterienzahlen im Reinwasserablauf auf die vorgeschriebenen Werte zurückzuführen, nötig.

Vorzugsweise wird für die Denitrifikation der Stamm "Micrococcus denitrificans" eingesetzt. Es handelt sich um einen autotrophen Stamm, der für seinen Stoffwechsel Wasserstoff benötigt. Die Wachstumsrate kann durch entsprechende Dosierung von Wasserstoff reguliert bzw. gebremst werden. Seinen Kohlenstoffbedarf deckt der Stamm aus der im Wasser gelösten Kohlensäure, und es findet nur ein Nitratabbau statt. Hierdurch ist die Bildung von unerwünschten Reaktionsnebenprodukten, wie z.B. Aldehyde, ausgeschlossen.

Je nach Erfordernis kann die biologische Denitrifikation zweistufig durchgeführt werden, um die vorgeschriebenen Nitratgehalte im Reinwasserablauf einzustellen.

Sofern das nitratbelastete Rohwasser erhöhte Konzentrationen an Sauerstoff aufweist, kann nennenswert an Reaktorvolumen bei der biologischen Denitrifikation eingespart werden, wenn in einer Vorstufe eine katalytische Desoxidation des Wassers durch Oxidation von zudosiertem Wasserstoff durchgeführt wird.

Zur Optimierung des Prozesses ist es notwendig, den pH-Wert des Wassers in der biologischen Denitrifikation auf einen Bestwert einzustellen. Dies geschieht vorteilhafterweise durch Zudosierung von Säure, z.B. Salzsäure.

Die erfindungsgemäße Vorrichtung zur Durchführung des Verfahrens besteht in ihrer einfachsten Form aus einem Reaktorbehälter mit einem Biokatalysatorbett, einer Wasserzuleitung und einer Wasseraustrittsleitung sowie einer Wasserstoffzumischleitung mit einem Umwälzkreislauf zwischen dem Wasserauslauf und dem Wassereinlauf zum Biokatalysatorbett, um die Denitrifikationsrate zu regulieren.

Mit diesem Umwälzkreislauf kann eine pH-Werteinstell-und -meßvorrichtung verbunden sein, die vorzugsweise eine Säuredosierpumpe aufweist.

Je nach der Nitratkonzentration im Rohwasser einerseits und dem zugelassenen Restnitratgehalt im Reinwasser können zwei oder mehr in Reihe geschaltete Reaktionsbehälter vorgesehen werden.

Die Umsetzung des im Rohwasser enthaltenen Sauerstoffs erfolgt in einem, der biologischen Denitrifikation vorgeschalteten Reaktionsbehälter mit einem Katalysatorbett, einer Wasserstoffzudosierung sowie einer Wasserzu-und -austrittsleitung.

Um die Anlage in einfacher Weise an unterschiedlich nitratbelastetes Rohwasser mit unterschiedlich hohen Konzentrationen an Sauerstoff anpassen zu können, sind vorteilhaferweise den oder die Reaktionsbehälter der biologischen Denitrifikation und/oder den Reaktionsbehälter der katalytischen Desoxidation überbrückende Umgehungsleitungen mit darin angeordneten Ventilen vorgesehen.

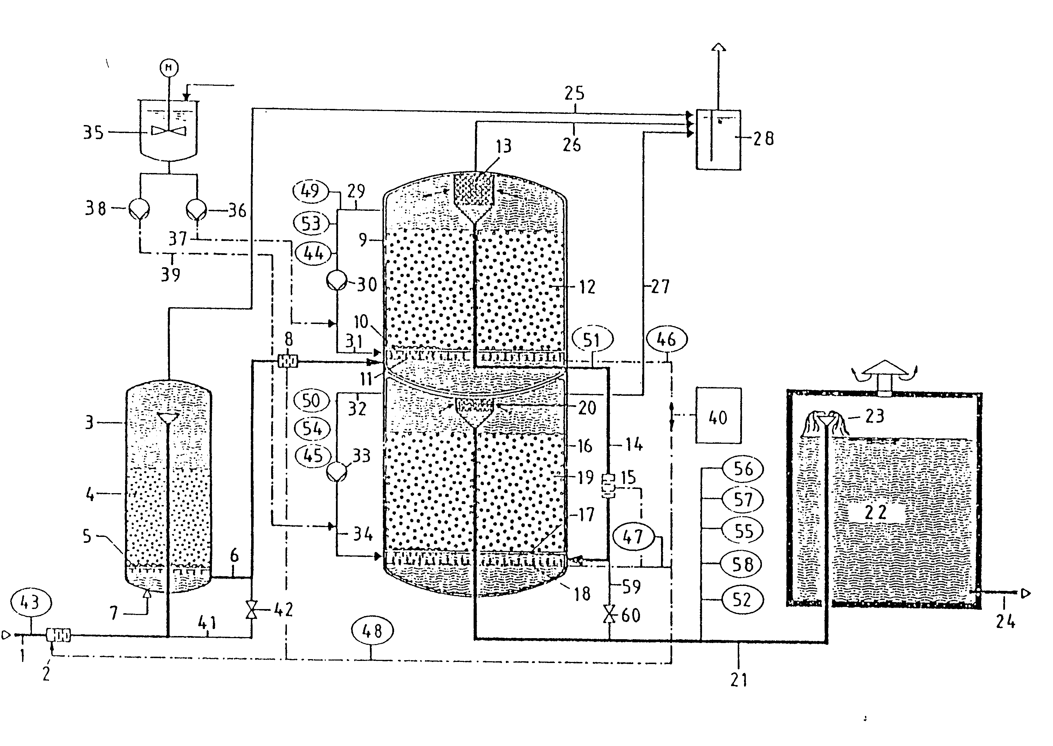

Die Erfindung wird nachstehend anhand eines in der Zeichnung schematisch dargestellten Ausführungsbeispiels des näheren erläutert.

Das zu behandelnde Rohwasser gelangt über eine Zuleitung 1 und einen statischen Mischer 2, über den von einer Wasserstoffdosiervorrichtung 40 Wasserstoff zudosiert wird, in einen Reaktionsbehälter 3. In diesem Reaktionsbehälter 3 ist ein edelmetallhaltiges Katalysatorbett 4, das auf einem Auflageboden 5 ruht, angeordnet. Als Katalysator werden z.B. Palladium oder andere Edelmetalle, verwendet. Der Reaktionsbehälter 3 ist als geschlossener Kessel ausgeführt, der für einen Betriebsdruck von etwa 3 bar bemessen ist. Das zugeführte Wasser tritt aus der Zuleitung 1 oberhalb des Katalysatorbettes 4 im Reaktionsbehälter 3 aus, fließt durch das Katalysatorbett 4 hindurch nach unten und wird über eine Austrittsleitung 6 abgeführt. Das Katalysatorbett 4 kann über einen besonderen Wasseranschluß 7 durch den Auflageboden 5 hindurch in an sich bekannter Weise zur zeitweisen Auflockerung rückgespült werden.

Diese Austrittsleitung 6 führt über einen weiteren statischen Mischer 8 für Wasserstoff in einen Reaktionsbehälter 9, der die erste Stufe der biologischen Denitrifikation bildet. Im Reaktionsbehälter 9 ist im unteren Bereich ein Auflageboden 10 für ein Biokatalysatorbett 12 vorgesehen, durch den das zu denitrifizierende Rohwasser geleitet wird. Am oberen Ende des Reaktionsbehälters 9 trifft das teilweise denitrifizierte Wasser durch eine Siebvorrichtung 13 in eine Austrittsleitung 14 ein, durch die es in einen Reaktionsbehälter 16, der die zweite Stufe der biologischen Denitrifikation bildet, gelangt. Der Reaktionsbehälter 16 ist in gleicher Weise wie der Reaktionsbehälter 9 aufgebaut und weist einen Auflageboden 17 mit darüber angeordnetem Biokatalysatorbett 19 auf. Auch dieser Reaktionsbehälter 16 wird vom zu denitrifizierenden Wasser von unten nach oben durchströmt und verläßt den Reaktionsbehälter 16 über eine Siebvorrichtung 20 und eine Austrittsleitung 21. Grundsätzlich besteht auch die Möglichkeit (im Schema nicht dargestellt), durch entsprechende Anordnung der Eintrittsleitung 11 und der Austrittsleitungen 14 und 21 an den Reaktionsbehältern 9 bzw. 16 den Wasserstrom aus dem oberen Raum der Reaktionsbehälter 9 bzw. 16 in deren unteren Teil zu leiten. Die Austrittsleitung 21 führt in einen Reinwassersammelbehälter 22, in dem ein plätschernder Überlauf 23 zur Sauerstoffanreicherung des denitrifizierten Wassers vorgesehen ist. Über eine Leitung 24 wird das behandelte Wasser an die Wasserversorgung abgegeben.

Zur Regelung der Denitrifikationsrate sind beide Denitrifikationsstufen mit einem eigenen, regelbaren Umlaufsystem ausgerüstet. Das Umlaufsystem des Reaktionsbehälters 9 besteht aus einer Leitung 29 stromabwärts oder stromaufwärts vom Biokatalysatorbett 12, eine Umwälzpumpe 30 und einer unterhalb des Auflagebodens 10 mündenden Leitung 31. Das Umlaufsystem für den Reaktionsbehälter 16 besteht entsprechend aus einer Leitung 32, einer Umwälzpumpe 33 und einer Leitung 34. In jeder Leitung 29 bzw. 32 sind ein pH-Wertmeßgerät 49,50 ein Wasserstoffgehaltmeßgerät 53,54 sowie ein Durchflußmesser 44,45 angeordnet.

Die pH-Wertmeßgeräte 49,50 dienen dazu, den pH-Wert in den Reaktionsbehältern 9 und 16 einzustellen. Dies geschieht mit Hilfe von Dosierpumpen 36,38, die über Leitungen 37,39 mit den Leitungen 31,34 verbunden sind. Mit Hilfe der Dosierpumpen 36,38 wird Säure, z.B. Salzsäure aus einem Behälter 35 entnommen und in der benötigten Menge den Reaktionsbehältern 9, 16 zugeführt. Eine zusätzliche Überwachung des pH-Wertes des Reinwassers erfolgt in der Leitung 21 mit Hilfe eines pH-Wertmeßgeräts 52.

Die Wasserstoffgehaltmeßgeräte 53, 54 dienen dazu, die Wasserstoffzufuhr zu den Mischern 2,8 und 15 über eine Wasserstoffdosiervorrichtung 40 zu regeln.

Gegebenenfalls kann die Wasserstoffzufuhr auch direkt in den Reaktionsbehälter 9 über ein Verteilersystem 11 und in den Reaktionsbehälter 16 über ein Verteilersystem 18 erfolgen. In der Zuleitung 1, den Leitungen 29, 32 und den von der Wasserstoffdosiervorrichtung 40 abgehenden Leitungen sind zusätzlich Durchflußmesser 43 bis 48 angeordnet, mit Hilfe derer der Durchsatz durch diese Leitungen bestimmt wird.

Zwischen der Austrittsleitung 14 aus dem Reaktionsbehälter 9 und der Austrittsleitung 21 aus dem Reaktionsbehälter 16 ist eine Umgehungsleitung 59 mit einem Ventil 60 vorgesehen, so daß ein mehr oder weniger großer Teilstrom des den ersten Reaktionsbehälter durchfließenden Wassers direkt dem Reinwassersammelbehälter 22 zugeführt werden kann, ohne den Reaktionsbehälter 16 zu durchlaufen. Die durch die Umgehungsleitung 59 strömend Wassermenge ist abhängig von der Nitratbelastung des Rohwassers und dem zulässigen Restnitratgehalt im Reinwasser.

Dieser Restnitratgehalt im Reinwasser wird mit Hilfe einer Meßvorrichtung 56 für den $NO_3$-Gehalt, einer Meßvorrichtung 57 für den $NO_x$-Gehalt, und einer Meßvorrichtung 58 für den $NO_2$-Gehalt bestimmt. Zusätzlich ist an dieser Stelle noch ein weiterer Wasserstoffgehaltmesser 55 vorgesehen.

Von den Reaktionsbehältern 3,9,16 führen Gasleitungen 25, 26,27 über eine Wasservorlage 28 nach außen, durch die die Reaktionsbehälter 3, 9, 16 entlüftet werden.

## Ansprüche

1. Verfahren zum Entfernen von Nitrat aus Oberflächen-und Grundwasser, insbesondere Trinkwasser, mit Hilfe der biologischen Denitrifikation, gekennzeichnet durch die Verwendung eines Biokatalysators mit immobilisierten Zellen, die in einem Gel oder Polymer vernetzt sind.

2. Verfahren nach Anspruch 1, gekennzeichnet durchdie Verwendung eines Biokatalysators aus einem Kern mit immobilisierten Zellen, die in einem Gel oder Polymer vernetzt sind und der mit einer zusätzlichen zellfreien, für Zellen undurchlässigen Schutzschicht, bestehend aus einem Gel oder einem Polymer, umschlossen ist.

3. Verfahren nach Anspruch 1 oder 2, dadurch gekennzeichnet, daß vor der biologischen Denitrifikation eine katalytische Desoxidation des Wassers durch Oxidation von zudosiertem Wasserstoff durchgeführt wird.

4. Verfahren nach Anspruch 1, 2 oder 3, gekennzeichnet durch die Verwendung des Stammes "Micrococcus denitrificans" im Biokatalysator.

5. Verfahren nach Anspruch 1, 2, 3 oder 4, dadurch gekennzeichnet, daß die biologische Denitrifikation zwei oder mehrstufig durchgeführt wird.

6. Verfahren nach einem oder mehreren der Ansprüche 1 bis 5, dadurch gekennzeichnet, daß der pH-Wert des Wassers in der biologischen Denitrifikation durch Zudosierung von Säure eingestellt wird.

7. Vorrichtung zur Durchführung des Verfahrens nach Anspruch 1 oder 2 aus einem Biokatalysatorbett, einer Wasserzuleitung und einer Wasseraustrittsleitung sowie einer Wasserstoffzumischleitung, dadurch gekennzeichnet, daß zwischen dem Wasserauslauf und dem Wassereinlauf bzw. -(19) ein Umwälzkreislauf (29, 30, 31 bzw. 32, 33, 34) angeordnet ist.

8. Vorrichtung nach Anspruch 7 zur Durchführung des Verfahrens nach Anspruch 5, gekennzeichnet durch eine mit dem Umwälzkreislauf (29, 30, 31 bzw. 32, 33, 34) verbundene pH-Wert-Einstell-und -meßvorrichtung (35, 36, 37, 49 bzw. 35, 38, 39, 50).

9. Vorrichtung nach Anspruch 8, gekennzeichnet durch eine Salzsäure-Dosierpumpe (36 bzw. 38).

10. Vorrichtung nach Anspruch 7, 8 oder 9 zur Durchführung des Verfahrens nach Anspruch 3, gekennzeichnet durch zwei oder mehr in Reihe geschaltete Reaktionsbehälter (9, 16).

11. Vorrichtung nach Anspruch 7, 8, 9 oder 10 zur Durchführung des Verfahrens nach Anspruch 4, gekennzeichnet durch einen der biologischen Denitrifikation vorgeschalteten Reaktionsbehälter (3) mit einem Katalysatorbett (4), einer Wasserstoffzudosierung (2) sowie einer Wasserzu-und -austrittsleitung (1, 6).

12. Vorrichtung nach Anspruch 10 oder 11, gekennzeichnet durch den oder die Reaktionsbehälter (16) der biologischen Denitrifikation und/oder den Reaktionsbehälter (3) der katalytischen Desoxidation überbrückende Umgehungsleitungen (41; 59) mit darin angeordneten Ventilen -(42; 60).

0 204 273